Europäisches Patentamt

(19) European Patent Office

Office européen des brevets

(11) Publication number: **0 010 389**
**B1**

(12) **EUROPEAN PATENT SPECIFICATION**

(45) Date of publication of patent specification: **15.06.83**

(51) Int. Cl.³: **A 61 F 5/01, A 61 F 13/06**

(21) Application number: **79302128.8**

(22) Date of filing: **05.10.79**

(54) Dynamic patellar brace.

(30) Priority: **06.10.78 US 949121**

(43) Date of publication of application:
**30.04.80 Bulletin 80/9**

(45) Publication of the grant of the patent:
**15.06.83 Bulletin 83/24**

(84) Designated Contracting States:
**DE FR GB IT NL SE**

(56) References cited:
**US - A - 3 804 084**
**US - A - 3 831 467**
**US - A - 3 926 186**
**US - A - 4 084 584**

(73) Proprietor: **Palumbo, Pasquale M.**
**906 Frome Lane**
**McLean, Virginia 22101 (US)**

(72) Inventor: **Palumbo, Pasquale M.**
**906 Frome Lane**
**McLean, Virginia 22101 (US)**

(74) Representative: **Allen, Oliver John Richard et al,**
**Lloyd Wise, Tregear & Co. Norman House 105-**
**109 Strand**
**London, WC2R 0AE (GB)**

Courier Press, Leamington Spa, England

## Dynamic patellar brace

The present invention relates to knee support devices, and more particularly to a brace or splinting device intended to provide dynamic bracing for and to stabilize the patella to prevent patellar subluxation during all normal degrees of knee flexion and motion.

It is well-known that loosely ligamented individuals, as well as individuals with certain peculiar anatomic features of certain components of the knee, frequently develop various pathological problems with their knees, particularly when these individuals are active in physically strenuous activities, such as athletic activities. The most commonly occurring problems relate to stretching or tearing of the various knee ligaments, injury to the cartilage (meniscal) and articular surfaces of the knee joint, and fractures. Patellar subluxation or abnormal and undesirable movement of the patella, laterally, relative to its normal up and down movement in the vertical track defined by the trochlea can precipitate the onset of chondromalacia or aggravate existing chondromalacia of the patella, as well as cause diagnostic problems of the knee.

Subluxation of the patella can be caused by certain developmental abnormalities of the skeletal components of the knee and/or the presence of musculoligamentous laxity or dysplasia. The patella may leave its normal, vertical tracking groove as a result of abnormal vector forces and/or by passive lateral or rotary forces. The abrupt abnormal lateral displacement of the patella from its groove during any weight bearing activity (such as running, stair climbing, etc.) frequently results in immediate, temporary disability (such as buckling of the knee), causing a subjective sensation in the knee similar to that caused by other unrelated pathological conditions within the knee.

The sensation of pain and/or imminent buckling of the knee results in apprehension and restriction of certain weight bearing activities such as athletic endeavours. The resultant increased abnormal traction forces on the peripatella soft tissues frequently lead to inflammatory changes of the retinaculae, patellar ligament and/or tendon (tendinitis).

Furthermore, the repetitive, abnormal lateral excursions which cause abnormal shearing forces, frequently lead to early, accelerated and progressive degenerative changes (chondromalacia) of the patella and femoral condyles.

As noted above, problems peculiar to the patella compromise only a portion of all common physiological problems of the knee and several, unrelated or partially related problems may occur simultaneously, particularly in individuals having loose ligaments or when engaged in relatively strenuous activities involving the knee. Physicians generally, and Orthopaedic Surgeons in particular, have only recently begun to fully appreciate the frequency and importance of patellar subluxation in the context of overall knee problems. Accordingly, the diagnosis of patella subluxation may be difficult to ascertain using conventional techniques, especially because other problems with the knee frequently cause subjective symptoms similar to those of subluxation of the patella.

Not only may the proper diagnosis of patellar subluxation, particularly in its milder form, be difficult using conventional techniques, but even when properly diagnosed, the preferred treatment may be somewhat limited. For example, young children still in the active bone growth phase of life frequently are relatively loosely ligamented and suffer from various degrees of patellar subluxation. However, it is well recognized that it is preferable to avoid or delay corrective surgery for such individuals, if at all possible, until such individuals reach a more physiologically opportune age when their growth plates have closed.

Others have devoted attention and proposed various knee braces and supports directed to general problems of the knee. For example, Spiro, U.S. Patent No. 3,473,527; Lehman, U.S. Patent No. 3,804,084, and Moore, U.S. Patent No. 3,853,123, have proposed various knee support, brace and knee splinting devices intended to restrain the knee to prevent normal knee flexion or movement. Such devices are directed, generally, to the problem of immobilizing the knee as a whole, and do not provide dynamic patellar bracing during normal knee flexion and extension.

Nirschl, U.S. Patent No. 3,926,186, Stromgern, U.S. Patent No. 3,945,046, and Detty, U.S. Patent No. 4,084,584 propose other muscular and flexible knee supports. Nirschl's apparatus, however, is not designed to provide medial-lateral stabilization of the patella, and is inherently incapable of performing a dynamic bracing function for the patella. Stromgern's apparatus, on the other hand, is not concerned with patellar stabilization, but instead is directed to the general problem of providing stability to the medial knee ligament complex. The Detty arrangement provides a combination of a conventional elastic knee sleeve provided with an aperture over the patella and a patellar bracing pad attached thereto alongside the aperture. It is capable of providing a limited passive bracing action but it lacks means to dynamically control the action of the patellar bracing pad throughout the normal range of flexion and movement of the knee.

The requirement therefore is to provide an arrangement which will maintain proper lateral positioning of the means used to control the movement of the patella and to provide effective lateral medial pressure on the patella throughout the full normal range of knee flexion and movement without manual repositioning or readjustment when the brace is in use.

Thus an object of the present invention is to provide a dynamic patellar brace useful both for diagnosis and treatment of patellar subluxation, and certain physiological problems of the knee related to or aggravated by patellar subluxation. A further object of the invention is to provide a dynamic patellar brace capable of performing a bracing or splinting function for the patella during the full, normal range of knee flexion and movement.

According to the invention a dynamic patellar brace for preventing subluxation of the human patella throughout the normal range of flexion and movement of the knee comprises in combination an elastic sleeve adapted to receive a leg with the respective knee positioned substantially within said sleeve when the brace is in use, said sleeve being provided with an aperture over the patella and a patellar bracing pad positioned on said sleeve so as to provide lateral support for the patella during flexion and movement of the knee when the brace is in use, said bracing pad being on the outside of the leg characterised in that the said bracing pad is provided with a pair of supporting arm members which in their operative positions pass from the top and bottom respectively of the bracing pad over and below the patella and around the inside of the knee and which apply via said bracing pad medial pressure to the patella throughout the normal range of flexion and movement of the knee and a third arm member which passes from the said bracing pad at its median axis away from the patella and around the outside of the knee and which applies a counter force to said bracing pad to maintain it in position relative to the patella. Such a brace may be used to facilitate proper and positive diagnosis of patellar subluxation, particularly in its milder form when its clinical presentation simulates that of other pathological conditions of the knee, and it is particularly suitable for use with children having patellar subluxation but whose growth plates are still open, so as to delay or avoid the need for corrective surgery until a more physiologically opportune time is reached.

The preferred embodiment of the invention is relatively simple to put in place and will provide dynamic patellar bracing without need for constant adjustment or re-adjustment. It can be worn with minimal discomfort, without being unsightly, and without requiring the user to utilize crutches or to walk in an unnatural manner. It has a relatively simple construction, and is relatively simple to manufacture.

Hereinafter the invention is further described by way of example and with reference to the accompanying drawing, wherein:—

Figure 1 is a perspective view of the dynamic patellar brace according to the present invention positioned on the knee and ready to have the elastic arm and counterarm members thereof wrapped circumferentially about the knee;

Figure 2 is a perspective view similar to Figure 1 but showing the two elastic arm members circumferentially wrapped, and with the counterarm member ready to be circumferentially wrapped about the knee;

Figure 3 is a further perspective view similar to Figure 2 but showing all of the elastic arm and counterarm members wrapped and held in the wrapped position.

Figure 4 is a perspective view, partially fragmentary, illustrating further details of a patellar brace according to the present invention.

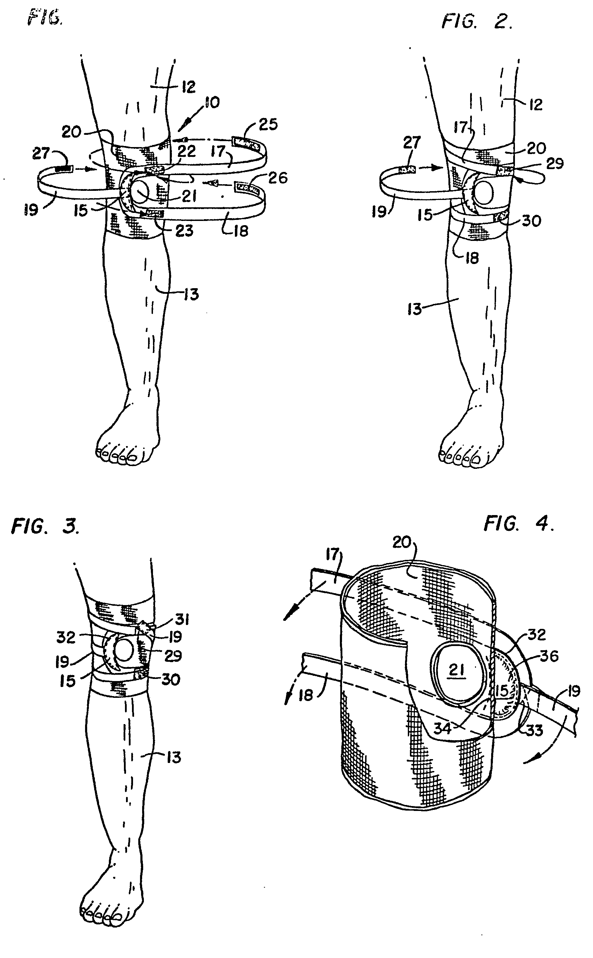

As shown in Figure 1, a dynamic patellar brace 10 according to the present invention is positioned on the knee between the upper portion 12 and lower portion 13 of the leg of the user (not shown). The patellar brace includes an elastic sleeve 20 through which the lower portion 13 of the user's leg has been inserted to facilitate positioning of a patellar bracing pad 15 laterally of the patella (not shown) of the user's knee. The elastic sleeve 20 includes an aperture 21 which is preferably positioned over or about the patella or knee cap by feel when the leg is inserted through the elastic sleeve 20.

As further shown in Figure 1, the dynamic patellar brace includes two elastic arm members 17, 18 adapted to be wrapped circumferentially in a first direction, counter-clockwise as shown in Figure 1, when the brace is in use. Each of the elastic arm members 17, 18 is attached to the patellar bracing pad 15, and arranged so that when circumferentially wrapped in the first direction about the user's knee when the brace is in use, the bracing pad 15 will thereby cause pressure to be applied medially to the user's patella.

The arm members 17 and 18 include thereon fastening and holding means 22 and 23, respectively, which preferably comprise Velcro strip means attached to the elastic arm members 17, 18 along the outer band surfaces thereof at positions near to the points at which the arm members 17, 18 are attached to the patellar bracing pad 15. Co-operating fastening and holding means 25, 26 associated respectively with the elastic arm members 17, 18 are attached to the inner band surfaces thereof, respectively, near the ends thereof furthest removed from the point of attachment to the patellar bracing pad 15.

The patellar brace 10 also includes a dynamic elastic counterarm 19 having one end thereof also attached to the patellar bracing pad 15, and the other end thereof adapted to be wrapped circumferentially in a direction opposite to the first direction, clockwise in Figure 1, about the user's knee. A fastening and holding means 27, preferably also a Velcro strip means, is attached to the inner band surface of the elastic arm member 19 as shown.

Figure 2 is a view similar to Figure 1, but illustrates the patellar brace 10 with the first

and second elastic arm members 17, 18 thereof circumferentially wrapped about the user's knee and fastened and held in the wrapped position. The fastening means 25 engages with the fastening means 22 on the arm member 17, and the fastening means 26 engages with the fastening means 23 on the arm member 18 to hold the arm members 17, 18 in the wrapped position. It is noted that the first arm member 17 is wrapped circumferentially about the knee above the patella, and the second arm member 18 is wrapped circumferentially about the knee below the patella. This arrangement causes each of the arm members 17 and 18 to exert pressure in the medial direction (i.e., toward the centre-line of the user) on the patellar bracing pad 15 to cause the patellar bracing pad 15 positioned laterally of the patella to thereby apply medial or inwardly directed pressure to the patella to prevent patellar subluxation.

As further shown in Figure 2, fastening or holding means 29, 30, preferably also Velcro strip means, are attached to the ends, respectively, of the arm members 17, 18, at the ends thereof furthest removed from the patellar bracing pad 15, on the outer band surfaces thereof. These fastening and holding means 29, 30 are adapted to co-operate with the fastening and holding means 27 on the inner surface of the counterarm member 19 as will be further described.

Figure 3 is a view similar to Figure 2, but showing the elastic counterarm member 19 now circumferentially wrapped in the clockwise direction with the end thereof furthest from the patellar bracing pad 15 fastened and held by co-operation between the fastening means 27 and the fastening means 29 previously described. It is noted it would be possible to fasten the end of the counterarm member 19 circumferentially wrapped about the knee by co-operation between the fastening means 27 and the fastening means 30 associated with the lower arm member 18 instead of with the fastening means 29 associated with the upper arm member 19. In either case, the counterarm member 19 serves to dynamically stabilize the position of the patellar bracing pad on the knee.

In a preferred embodiment, either of the fastening means 29 or 30 is capable of co-operating with the fastening means 27 to hold the counterarm member 19 in the wrapped position. This arrangement facilitates wrapping and fastening of the counterarm member as the person initially applying the brace to the user may choose to fasten the end of the counterarm member 19 furthest from the patellar bracing pad either to the upper or to the lower arm member 18 or 19. The precise point at which the counterarm member is fastened and held generally makes little or no difference to the user. This arrangement also permits the use of a single brace for either the left or the right knee of a user as it allows the upper and lower portions of the brace to be mirror images of each other.

It is also seen from Figure 3 that the fastening or holding means 27, preferably a Velcro strip means, is fastened to the end of the counterarm 19 furthest from the patellar bracing pad 15 by stitching means 31. The other fastening or holding means 22, 23, 25, 26, 29 and 30 may also be fastened to the elastic band members by similar stitching means (not shown) or by other appropriate adhesive or epoxy means.

Figure 4 illustrates further details of the elastic sleeve member 20 and the connection and attachment thereto of the patellar bracing pad 15, and the attachment of the arm members 17 and 18 and the counterarm member 19 thereto and to the pad 15. As shown in Figure 4, the elastic sleeve member 20 preferably comprises a sleeve of elastic material having an approximately circular aperture 21 therein. The lower portion 13 of the leg of the user of the patellar brace may be inserted through the elastic sleeve 20 so that his or her knee cap or patella is positioned within the sleeve 20 in registration with the circular aperture 21. This can be readily accomplished by manually feeling the position of the patella or the knee cap, and making sure that the patella is initially moved to its normal up and down vertical track defined by the trochlea. Then, by ensuring that the aperture 21 of the sleeve 20 is positioned over the patella, the user of the brace automatically obtains proper positioning of the patellar bracing pad 15 laterally of his or her patella. The user, of course, must be sophisticated enough to realize that the patellar bracing pad 15 is to be positioned laterally of the patella. Thus, the user must insert his leg through a different end of the elastic sleeve 20 depending upon whether the brace 10 of Figures 1—3 is to be used on his or her left or right knee. It would, of course, be possible to mark the ends of the sleeve 20 for left knee or right knee use.

In a preferred embodiment the diameter of the elastic sleeve member and the elastic material and forces thereof are selected so as to not unduly constrict the blood flow within the leg or knee of the user. Preferably, a series of different sized braces must be provided to accommodate different users. Although a primary purpose of the sleeve 20 and the aperture 21 therein is to permit a relatively unskilled user of the brace to achieve accurate alignment of the patellar bracing pad relative to the patella, the elastic sleeve member 20 also inhibits sliding movement of the brace about the knee. However, it is the arrangement, wrapping, and fastening of the arm 17, 18 and counterarm 19 members circumferentially about the knee which primarily causes the patellar bracing pad 15 to be maintained in position laterally of the patella and to provide medially directed pressure thereto throughout the normal range of knee flexion and movement.

It should also be noted that the patellar bracing pad 15 is arcuately shaped, concave toward the aperture 21 which is adapted to be positioned over the patella when the patella is initially in its normal vertical track defined by the trochlea when the brace is first applied. It is also seen from Figure 4 which shows the underside of the patellar bracing pad 15 that the pad 15 preferably has a thickness or elevation 36 to it, and is preferably formed from a partially resilient, padding material adapted to permit the pad 15 to contour itself to the patella against which it applies medially directed forces. The patellar bracing pad 15 is also preferably attached to the sleeve 20 by stitching means 34.

As further shown in Figure 4, the upper and lower arm members 17 and 18 and the counterarm member 19 may also be attached to the patellar bracing pad 15 by appropriate stitching means 32, 33. The upper and lower elastic arm members 17 and 18 can be constructed from a single U-shaped piece of elastic band or rubber material and attached by stitching means 32, 33 to the bracing pad 15. This arrangement facilitates development of uniform medially directed forces in the bracing pad 15 when the elastic arm members 17, 18 are circumferentially wrapped about the knee as previously described. The elastic counterarm 19 is also preferably constructed from an elastic band or rubber material and is preferably attached to the bracing pad 15 at the vertical midpoint thereof by stitching means 33.

It may also be seen that the arcuately shaped bracing pad 15, concave towards aperture 21 (i.e., towards the patella) will tend to dynamically confine the patella to its normal up and down movement in the vertical track defined by the trochlea. In particular, during full flexion or bending of the knee, the upper and lower portions of the bracing pad 15 will bend with the knee and provide pressure surfaces to continuously provide medial pressure against the patella to prevent subluxation in the lateral direction. As previously noted, subluxation of the patella can only occur in the lateral or outwardly moving direction on each knee, and it is only necessary, therefore, to provide a dynamic, lateral restraint to prevent such subluxation.

It may now be seen that the dynamic patella brace 10 of the present invention may be advantageously used to prevent patellar subluxation in the user during all normal degrees of knee flexion and movement. Unlike prior art devices, the combination of the elastic sleeve 20, the circumferentially wrapped arms 17, 18 and the circumferentially wrapped counterarm 19 cause the patellar bracing pad 15 to be maintained in proper position laterally of the patella during all normal degrees of knee flexion and motion and to apply medially directed pressure to the patella of the user to prevent subluxation.

As noted previously, a dynamic patellar brace according to the present invention may be advantageously employed for diagnosis of patellar subluxation difficulties of the knee, particularly when patellar subluxation presents in its milder form and/or its symptoms simulate those of other pathological conditions of the knee. For purposes of diagnosis, the patellar brace may be applied by the physician or Orthopaedic Surgeon to the patient suspected of having a patellar subluxation problem with instructions to the patient to wear the brace for a relatively short period of time and to perform a series of diagnostic test exercises. Such test exercises may include ascending and descending stairs or pivoting on the involved extremity while running. At the conclusion of such series of diagnostic test exercises, the patient will be requested to state whether he or she noticed reduced pain or increased stability of the knee during such exercises. If the patient reports such an improvement, the physician may conclude that patellar subluxation is at least a contributing factor, if not the causative agent, in the patient's knee difficulties or complaints.

In cases in which patellar subluxation problems are diagnosed, the dynamic patellar brace according to the present invention may then also be used for treatment purposes. For example, in young children still in the active bone growth phase of life, the wearing of a dynamic patellar brace according to the present invention will prevent patellar subluxation and related problems, and allow corrective surgery to be postponed or avoided. It is well known that surgery is preferably avoided, if at all possible, in relatively young children because disturbance of the growth plates in such children, which may accidentally occur, can result in uneven bone growth rates and possible deformity after surgery. It is also well known that relatively loose ligaments occur more often in younger persons and that the musculoligamentous structures tend to become tighter with advancing age. Thus the patellar brace, according to the present invention, may be advantageously used in younger persons in that it may obviate the need for future surgical intervention in certain milder forms.

It is also believed that dynamic patellar braces according to the present invention are useful for treating patellar subluxation by simply preventing further stretching of the ligaments. With repeated subluxation, the medial ligament of the patellae (retinaculum) becomes stretched and the lateral ligament (retinaculum) tightens, tending to more readily permit further subluxation occurrences. If the patellar brace according to the present invention is utilized, however, the patella will be confined to its normal up and down vertical track defined by the trochlea, and, therefore, prevent the development of or stretch an already tight lateral retinaculum. Thus, the need for corrective surgery may be avoided.

It is also believed that the patellar stabilizing brace may be useful in certain forms of isolated patellofemoral chondromalacia by changing opposing contact or pressure points which are frequently painful.

**Claims**

1. A dynamic patellar brace (10) for preventing subluxation of the human patella throughout the normal range of flexion and movement of the knee comprising in combination an elastic sleeve (20) adapted to receive a leg with the respective knee positioned substantially within said sleeve (20) when the brace (10) is in use, said sleeve being provided with an aperture (21) over the patella and a patellar bracing pad (15) positioned on said sleeve (20) so as to provide lateral support for the patella during flexion and movement of the knee when the brace (10) is in use, said bracing pad (15) being on the outside of the leg characterised in that the said bracing pad (15) is provided with a pair of supporting arm members (17, 18) which in their operative positions pass from the top and bottom respectively of the bracing pad (15) over and below the patella and around the inside of the knee and which apply via said bracing pad (15) medial pressure to the patella throughout the normal range of flexion and movement of the knee, and a third arm member (19) which passes from the bracing pad (15) at its median axis away from the patella and around the outside of the knee and which applies a counter force to said bracing pad (15) to maintain it in position relative to the patella.

2. A dynamic brace according to Claim 1 characterised in that said arm members (17, 18 and 19) comprise elastic straps.

3. A dynamic brace according to Claim 1 or Claim 2 characterised in that said pair of arm members (17, 18) are of such length that they wrap completely around the leg and their free ends are secured to the patellar brace at the front of the knee.

4. A dynamic brace according to Claim 3 characterised in that the third arm member (19) is of such length that it wraps around the leg, and its free end is arranged to be secured above or below the patella.

5. A dynamic brace according to any preceding claim wherein the first said pair of supporting arm members (17, 18) comprise the legs of a unitary U-shaped member.

6. A dynamic brace according to any preceding claim characterised in that the patella bracing pad (15) is arcuate shaped and is arranged so that its concave edge applies pressure to the patella.

7. A dynamic brace according to Claim 6 characterised in that the patellar bracing pad (15) comprises an elongate pad arranged with its elongate dimension paralleled with the leg axis.

**Patentansprüche**

1. Dynamische Kniescheiben-(Pateller)-Stütze (10) zum Verhindern des Ausrenkens der menschlichen Kniescheibe im normalen Bereich von Beugen und Bewegung des Knies mit der Kombination einer elastischen Muffe (20) zur Aufnahme eines Beins mit dem entsprechenden Knie, im wesentlichen innerhalb der Muffe bei Gebrauch der Stütze, wobei diese Muffe (20) mit einer Öffnung (21) über der Kniescheibe versehen ist und ein Kniescheibenstützkissen (15) sich an der Muffe (20) befindet, so daß sich bei Gebrauch der Stütze (10) eine seitliche Stütze für die Kniescheibe während der Bewegung und der Beugung des Knies ergibt, und das Stützkissen (15) sich an der Außenseite des Beins befindet, dadurch gekennzeichnet, daß das Stützkissen (15) mit einem Stützenpaar (17, 18) versehen ist, die in ihren Arbeitslagen oben bzw. unten vom Stützkissen (15) über und unter der Kniescheibe und rund um die Innenseite des Knies herum verlaufen und über das Stützkissen (15) einen Mittendruck auf die Kniescheibe während des normalen Beugens und Bewegens des Knies bewirken, und daß eine dritte Stütze (19) vom Stützkissen (15) an dessen Mittelachse von der Kniescheibe weg und rund um die Außenseite des Knies herum verläuft und eine Gegenkraft an das Stützkissen anlegt, um es zur Kniescheibe ausgerichtet zu halten.

2. Stütze nach Anspruch 1, dadurch gekennzeichnet, da die Stützen (17, 18 und 19) elas elastische Streifen enthalten.

3. Stütze nach Anspruch 2, dadurch gekennzeichnet, daß das Stützenpaar (17, 18) so lang ist, daß sie das Bein vollständig umgeben und ihre freien Enden an der Vorderseite des Knies an der Kniescheibenstütze befestigbar sind.

4. Stütze nach Anspruch 3, dadurch gekennzeichnet, daß die dritte Stütze (19) so lang ist, daß sie das Bein umschlingt und und das freie Ende über oder unter der Kniescheibe befestigbar ist.

5. Stütze nach den vorhergehenden Ansprüchen, dadurch gekennzeichnet, daß das erste Stützenpaar (17, 18) die Schenkel eines gleichmäßigen U-förmigen Teils enthält.

6. Stütze nach den vorhergehenden Ansprüchen, dadurch gekennzeichnet, daß das Kniescheibenstützkissen (15) bogenförmig ist und so angeordnet ist, daß seine konkave Kante Druck an die Kniescheibe legt.

7. Stütze nach Anspruch 6, dadurch gekennzeichnet, daß das Kniescheibenstützkissen (15) ein längliches Kissen enthält, das mit seiner Längsabmessung zur Achse des Beins parallel verläuft.

**Revendications**

1. Dispositif de soutien dynamique pour rotule pour empêcher une subluxation de la rotule humaine dans le cours de la gamme normale de flexion et de mouvement du genou, comprenant en combinaison un manchon élastique 20 destiné à recevoir la jambe avec le genou respectivement, positionné sensiblement à l'intérieur dudit manchon 20 quand le soutien 10 est en service, ledit manchon étant pourvu d'une ouverture 21 au-dessus de la rotule et un bourrelet 15 de soutien de rotule positionné sur ledit manchon 20 de manière à fournir un support latéral pour la rotule durant la flexion et le mouvement du genou quand le soutien 10 est en service, ledit bourrelet 15 de soutien étant sur l'extérieur de la jambe, caractérisé en ce que ledit bourrelet 15 de soutien est pourvu d'une paire de pattes de support 17, 18 qui en position opérationnelle, passent à partir du haut et du bas respectivement du bourrelet 15 de soutien au-dessus et au-dessous de la rotule et autour de l'intérieur du genou et qui appliquent par l'intermédiaire dudit bourrelet 15 de soutien, une pression médiane sur la rotule au cours de la gamme normale de flexion et de mouvement du genou, et une troisième patte 19 qui passe à partir du bourrelet 15 de soutien en son axe médian depuis la rotule et autour de l'extérieur du genou et qui applique une contre-force audit bourrelet 15 de soutien pour le maintenir en position relative par rapport à la rotule.

2. Dispositif de soutien dynamique selon la revendication 1 caractérisé en ce que lesdites pattes 17, 18 et 19 comprennent des bandes élastiques.

3. Dispositif de soutien dynamique selon l'une quelconque des revendications 1 ou 2, caractérisé en ce que ladite paire de pattes 17, 18 est d'une longueur telle qu'elle s'enroule complètement autour de la jambe et que ses extrémités libres sont fixées au soutien de rotule sur le devant du genou.

4. Dispositif de soutien dynamique selon la revendication 3, caractérisé en ce que la troisième patte 19 est d'une longueur telle qu'elle s'enroule autour de la jambe et que son extrémité libre est disposée de manière à être fixée au-dessus ou au-dessous de la rotule.

5. Dispositif de soutien dynamique selon l'une quelconque des revendications précédentes, caractérisé en ce que la première paire de pattes de support 17, 18 est constituée par les branches d'une pièce unitaire en forme de U.

6. Dispositif de soutien dynamique selon l'une quelconque des revendications précédentes, caractérisé en ce que le bourrelet 15 de soutien de la rotule est en forme d'arc et est disposé de manière que son côté concave applique une pression sur la rotule.

7. Dispositif de soutien dynamique selon la revendication 6 caractérisé en ce que le bourrelet 15 de soutien de rotule comprend un bourrelet allongé disposé de manière que son bord allongé soit parallèle à l'axe de la jambe.

FIG.

FIG. 2.

FIG. 3.

FIG. 4.